# EUROPEAN PATENT SPECIFICATION

(11) **EP 2 700 945 B1**
(45) Date of publication and mention of the grant of the patent: **02.11.2016**
(21) Application number: 12774207.0
(22) Date of filing: 18.04.2012
(51) Int. Cl.: G01N 33/53, C12Q 1/68, G01N 33/15, G01N 33/50, G01N 33/564, C12N 15/09

(54) **USE OF MYELIN BASIC PROTEIN AS A NOVEL GENETIC FACTOR FOR RHEUMATOID ARTHRITIS**
VERWENDUNG DES BASISCHEN MYELINPROTEINS ALS NEUER GENETISCHER FAKTOR FÜR RHEUMATOIDE ARTHRITIS
UTILISATION D'UNE PROTÉINE BASIQUE DE MYÉLINE COMME NOUVEAU FACTEUR GÉNÉTIQUE POUR LA POLYARTHRITE RHUMATOÏDE

(30) Priority: 22.04.2011 JP 2011095625
(43) Date of publication of application: 26.02.2014
(60) Divisional application: 16189516.4
(73) Proprietor: Kyoto University, Kyoto-shi, Kyoto 606-8501 (JP)
(72) Inventor: MATSUDA, Fumihiko, Kyoto-shi Kyoto 606-8501 (JP); MIMORI, Tsuneyo, Kyoto-shi Kyoto 606-8501 (JP); OHMURA, Koichiro, Kyoto-shi Kyoto 606-8501 (JP); TERAO, Chikashi, Kyoto-shi Kyoto 606-8501 (JP)
(74) Representative: Tuxworth, Pamela M.
(86) International application number: PCT/JP2012/060426
(87) International publication number: WO 2012/144512

(56) References cited:
- JP-A- 2001 139 496
- JP-A- 2003 079 270
- JP-A- 2003 512 599
- JP-A- 2007 186 508
- JP-A- 2007 517 019
- JP-A- 2010 190 898
- NATALIA WEGNER ET AL: "Autoimmunity to specific citrullinated proteins gives the first clues to the etiology of rheumatoid arthritis", IMMUNOLOGICAL REVIEWS, vol. 233, no. 1, 1 January 2010 (2010-01-01), pages 34-54, XP55143800, ISSN: 0105-2896, DOI: 10.1111/j.0105-2896.2009.00850.x
- Illumina: "HumanHap550v3_A: probe Pr031450693", , 2 December 2009 (2009-12-02), XP002730690, Retrieved from the Internet: URL:http://www.ncbi.nlm.nih.gov/probe/?ter m=Pr031450693 [retrieved on 2014-10-07]
- ANTONIO JULIÀ ET AL: "Genome-wide association study of rheumatoid arthritis in the Spanish population: KLF12 as a risk locus for rheumatoid arthritis susceptibility", ARTHRITIS & RHEUMATISM, vol. 58, no. 8, 1 August 2008 (2008-08-01) , pages 2275-2286, XP55143540, ISSN: 0004-3591, DOI: 10.1002/art.23623
- ANDREEA IOAN-FACSINAY ET AL: "Marked differences in fine specificity and isotype usage of the anti-citrullinated protein antibody in health and disease", ARTHRITIS & RHEUMATISM, vol. 58, no. 10, 1 October 2008 (2008-10-01), pages 3000-3008, XP055143964, ISSN: 0004-3591, DOI: 10.1002/art.23763
- SUN YILONG ET AL: "Rearrangement and reactivation of the myelin basic protein locus in myelin-deficient (mld) mouse brain", JOURNAL OF NEUROCHEMISTRY, vol. 68, no. 2, 1997, pages 457-468, XP002730691, ISSN: 0022-3042
- BOIRE GILLES ET AL: "Anti-Sa antibodies and antibodies against cyclic citrullinated peptide are not equivalent as predictors of severe outcomes in patients with recent-onset polyarthritis", ARTHRITIS RESEARCH, CURRENT SCIENCE, LONDON, GB, vol. 7, no. 3, 17 March 2005 (2005-03-17), pages R592-R603, XP021011603, ISSN: 1465-9905, DOI: 10.1186/AR1719
- IOAN-FACSINAY ANDREEA ET AL: "Anti-cyclic citrullinated peptide antibodies are a collection of anti-citrullinated protein antibodies and contain overlapping and non-overlapping reactivities", ANNALS OF THE RHEUMATIC DISEASES, BRITISH MEDICAL ASSOCIATION, LONDON, GB, vol. 70, no. 1, 1 January 2011 (2011-01-01), pages 188-193, XP009180494, ISSN: 0003-4967 [retrieved on 2010-08-24]
- CHIKASHI TERAO ET AL: "Myelin Basic Protein as a Novel Genetic Risk Factor in Rheumatoid Arthritis-A Genome-Wide Study Combined with Immunological Analyses", PLOS ONE, vol. 6, no. 6, 17 May 2011 (2011-05-17), page e20457, XP055143432, ISSN: 1932-6203, DOI: 10.1371/journal.pone.0020457

### SEQUENCE LISTING

<110> Kyoto University
<120> Use of myelin basic protein as a novel genetic risk factor in
   rheumatoid arthritis
<130> FP-169PCT
<150> JP P2011-095625
   <151> 2011-04-22
<160> 2
<170> PatentIn version 3.1
<210> 1
   <211> 1602
   <212> DNA
   <213> Homo sapiens
<400> 1
   agtttcatat gagaaaaaga aataataagg ttttcagacc tctcatcttc cagttcattc      60
   gcctgtgaac tgagacagtg ttcaaaacac caaagtgaaa gaattcaaat ggtgacaaat     120
   aactcaaggg cactacaaac ccctcctttg ggttggtggg aagtggggga gcagggagca     180
   ctggggaagg gtacagggtc cccagagacc aggcctgttc tggaaccttc cacggacacc     240
   cccccaccaa cagggcaaca caagtgagcc ccccactgtg ggccaactcg gggcaaggac     300
   tcccctgatc ctttggataa tcgttcaggt aaattcccaa aggtcatggc tcacagatat     360
   tgggacccca gctctctacc tgtgaagatg gggtctgcag gagccaggca gaggggagcc     420
   ctgtggcagc tcttcctgtt gtggacaact ttctcctcct ggaacccatc tctcttcttg     480
   gctctcctga tagtttccct ctcctctcag ctatcagtca cctgggagga ttccatctcc     540
   tctgcataac aaaagatgcc actgacactc ttttccgcag gatgttgctg ctctgggcga     600
   tagtttcata tgagaaaaag aaataataag gttttcagac ctctcatctt ccagttcatt     660
   cgcctgtgaa ctgagacagt gttcaaaaca ccaaagtgaa agaattcaaa tggtgacaaa     720
   taactcaagg gcactacaaa cccctccttt gggttggtgg gaagtggggg agcagggagc     780
   actggggaag ggtacagggt ccccagagac caggcctgtt ctggaacctt ccacggacac     840
   ccccccacca acagggcaac acaagtgagc cccccactgt gggccaactc ggggcaagga     900
   ctcccctgat cctttggata atcgttcagg taaattccca aaggtcatgg ctcacagata     960
   ttgggacccc agctctctac ctgtgaagat ggggtctgca ggagccaggc agaggggagc    1020
   cctgtggcag ctcttcctgt tgtggacaac tttctcctcc tggaacccat ctctcttctt    1080
   ggctctcctg atagtttccc tycctctcag ctatcagtca cctgggagga ttccatctcc    1140
   tctgcataac aaaagatgcc actgacactc ttttccgcag gatgttgctg ctctgggcga    1200
   tgtcctaggg gagaagggag ctttgagctt ccaggattca agaggaaaac agctaccctc    1260
   gggagacaga gtctcagcca gggccatctg cagagccaga catcttctac acagcctgag    1320
   acaattccaa cctgtgttaa taaattgcag cctcacccag cgggcatcct ctcagaaact    1380
   gagatctcag tgggatgtgt taggagctgc cagccactca cagagacatg gattcatcac    1440
   agagggtggc tgtctcccag caatcgctct gcgagttaca tttggatggg tctgcagagc    1500
   caggtgtttt ggaaggcaga gctctttgtg ggataaagct ggactgggtg gagccggtat    1560
   tagggggaca cctggaccct acagccttgg cgatactgag tg                       1602
<210> 2
   <211> 1001
   <212> DNA
   <213> Homo sapiens
<400> 2
   tattgggacc ccagctctct acctgtgaag atggggtctg caggagccag gcagagggga     60
   gccctgtggc agctcttcct gttgtggaca actttctcct cctggaaccc atctctcttc    120
   ttggctctcc tgatagtttc cctccctctc agctatcagt cacctgggag gattccatct    180
   cctctgcata acaaaagatg ccactgacac tcttttccgc aggatgttgc tgctctgggc    240
   gatgtcctag gggagaaggg agctttgagc ttccaggatt caagaggaaa acagctaccc    300
   tcgggagaca gagtctcagc cagggccatc tgcagagcca gacatcttct acacagcctg    360
   agacaattcc aacctgtgtt aataaattgc agcctcaccc agcgggcatc ctctcagaaa    420
   ctgagatctc agtgggatgt gttaggagct gccagccact cacagagaca tggattcatc    480
   acagagggtg gctgtctccc rgcaatcgct ctgcgagtta catttggatg ggtctgcaga    540
   gccaggtgtt ttggaaggca gagctctttg tgggataaag ctggactggg tggagccggt    600
   attaggggga cacctggacc ctacagcctt ggcgatactg agtgggagag gtttgcagca    660
   ggtgccccaa atcaaatctt ttttttaaaa aaatacagta tttaaatatt tgcttatgct    720
   tgtctcaacc ctgggaagtg gggaaggaca cccctgtctc tccctctatg tgagccctcc    780
   ccgtagcttc tcttgctggc ctgtgggctc tacagcccca gggagtcctg cttctgtcct    840
   gagctcttca tacccatcac tgctctgagt gtgcttttgt aattttgttg gtctcctgat    900
   tagactgtga ggtattctga ggacagacac aaggacttcc tttttttttt tttttttttt    960
   tacaataata gcaataagaa gaaaattagt aacaccaaac a                        1001

## Description

### TECHNICAL FIELD

The present invention relates to use of myelin basic protein as a novel genetic factor for rheumatoid arthritis.

### BACKGROUND ART

Rheumatoid arthritis (hereinafter, abbreviated to "RA") is a systemic disease associated with various symptoms including joint pain, dysfunction and deformity, as well as interstitial pneumonia and scleritis. RA is the most common cause of adult chronic inflammatory arthritis, affecting 0.5-1% of the adult population worldwide. Both genetic and environmental factors have been implicated in the development of RA (Non-Patent Documents Nos. 1 and 2). Although the polymorphisms of HLA as a human major histocompatibility antigen are the strongest genetic factors for RA, these can account for only 30-50% of the total genetic factors (Non-Patent Document No. 3). To date, various novel genetic factors have been identified by genome-wide, comprehensive analyses (GWAS: genome-wide association study) in case-control groups using markers (mainly SNPs) (Non-Patent Documents Nos. 4-21). However, even with all the novel genetic factors being considered together, only a small part of the genetic factors for RA can be explained. Besides, ethnic specificity is very strong in these genetic factors and many of them are risk factors limited to Europeans and Americans while equally many are risk factors limited to Asians (Non-Patent Documents Nos. 22-26, 12 and 17).

Conventionally, detection of autoantibodies in sera from RA patients has been performed by ELISA techniques, among which anti-CCP antibody detection ELISA is the most common method used today. Anti-CCP antibodies are autoantibodies excellent in both sensitivity and specificity to RA patients (Non-Patent Document Nos. 27 and 28). However, they are antibodies not against a specific protein but against a plurality of citrullinated proteins and, moreover, no antigens have been identified for commercialized anti-CCP antibodies. Further, few autoantibodies other than anti-CCP antibody have so far been known to be useful in diagnosing anti-CCP antibody-negative RA patients.

### PRIOR ART LITERATURE

### Non-Patent Documents

Non-Patent Document No. 1: Firestein GS (2003) Evolving concepts of rheumatoid arthritis. Nature 423: 356-361.
Non-Patent Document No. 2: MacGregor AJ, Snieder H, Rigby AS, Koskenvuo M, Kaprio J, et al. (2000) Characterizing the quantitative genetic contribution to rheumatoid arthritis using data from twins. Arthritis Rheum 43: 30-37.
Non-Patent Document No. 3: Deighton CM, Walker DJ, Griffiths ID, Roberts DF (1989) The contribution of HLA to rheumatoid arthritis. Clin Genet 36: 178-182.
Non-Patent Document No. 4: Begovich AB, Carlton VE, Honigberg LA, Schrodi SJ, Chokkalingam AP, et al. (2004) A missense single-nucleotide polymorphism in a gene encoding a protein tyrosine phosphatase (PTPN22) is associated with rheumatoid arthritis. Am J Hum Genet 75: 330-337.
Non-Patent Document No. 5: Plenge RM, Seielstad M, Padyukov L, Lee AT, Remmers EF, et al. (2007) TRAF1-C5 as a risk locus for rheumatoid arthritis--a genomewide study. N Engl J Med 357: 1199-1209.
Non-Patent Document No. 6: Remmers EF, Plenge RM, Lee AT, Graham RR, Hom G, et al. (2007) STAT4 and the risk of rheumatoid arthritis and systemic lupus erythematosus. N Engl J Med 357: 977-986.
Non-Patent Document No. 7: Raychaudhuri S, Remmers EF, Lee AT, Hackett R, Guiducci C, et al. (2008) Common variants at CD40 and other loci confer risk of rheumatoid arthritis. Nat Genet 40: 1216-1223.
Non-Patent Document No. 8: Plenge RM, Cotsapas C, Davies L, Price AL, de Bakker PI, et al. (2007) Two independent alleles at 6q23 associated with risk of rheumatoid arthritis. Nat Genet 39: 1477-1482.
Non-Patent Document No. 9: Thomson W, Barton A, Ke X, Eyre S, Hinks A, et al. (2007) Rheumatoid arthritis association at 6q23. Nat Genet 39: 1431-1433.
Non-Patent Document No. 10: Raychaudhuri S, Thomson BP, Remmers EF, Eyre S, Hinks A, et al. (2009) Genetic variants at CD28, PRDM1 and CD2/CD58 are associated with rheumatoid arthritis risk. Nat Genet 41: 1313-1318.
Non-Patent Document No. 11: Gregersen PK, Amos CI, Lee AT, Lu Y, Remmers EF, et al. (2009) REL, encoding a member of the NF-kappaB family of transcription factors, is a newly defined risk locus for rheumatoid arthritis. Nat Genet 41: 820-823.
Non-Patent Document No. 12: Stahl EA, Raychaudhuri S, Remmers EF, Xie G, Eyre S, et al. (2010) Genome-wide association study meta-analysis identifies seven new rheumatoid arthritis risk loci. Nat Genet 42: 508-514.
Non-Patent Document No. 13: Suzuki A, Yamada R, Chang X, Tokuhiro S, Sawada T, et al. (2003) Functional haplotypes of PADI4, encoding citrullinating enzyme peptidylarginine deiminase 4, are associated with rheumatoid arthritis. Nat Genet 34: 395-402.
Non-Patent Document No. 14: Tokuhiro S, Yamada R, Chang X, Suzuki A, Kochi Y, et al. (2003) An intronic SNP in a RUNX1 binding site of SLC22A4, encoding an organic cation transporter, is associated with rheumatoid arthritis. Nat Genet 35: 341-348.
Non-Patent Document No. 15: Kochi Y, Yamada R, Suzuki A, Harley JB, Shirasawa S, et al. (2005) A functional variant in FCRL3, encoding Fc receptor-like 3, is associated with rheumatoid arthritis and several autoimmunities. Nat Genet 37: 478-485.
Non-Patent Document No. 16: Suzuki A, Yamada R, Kochi Y, Sawada T, Okada Y, et al. (2008) Functional SNPs in CD244 increase the risk of rheumatoid arthritis in a Japanese population. Nat Genet 40: 1224-1229.
Non-Patent Document No. 17: Kochi Y, Okada Y, Suzuki A, Ikari K, Terao C, et al. (2010) A regulatory variant in CCR6 is associated with rheumatoid arthritis susceptibility. Nat Genet 42: 515-519.
Non-Patent Document No. 18: Kobayashi S, Ikari K, Kaneko H, Kochi Y, Yamamoto K, et al. (2008) Association of STAT4 with susceptibility to rheumatoid arthritis and systemic lupus erythematosus in the Japanese population. Arthritis Rheum 58: 1940-1946.
Non-Patent Document No. 19: Shimane K, Kochi Y, Yamada R, Okada Y, Suzuki A, et al. (2009) A single nucleotide polymorphism in the IRF5 promoter region is associated with susceptibility to rheumatoid arthritis in the Japanese population. Ann Rheum Dis 68: 377-383.
Non-Patent Document No. 20: (2007) Genome-wide association study of 14,000 cases of seven common diseases and 3,000 shared controls. Nature 447: 661-678.
Non-Patent Document No. 21: Orozco G, Sanchez E, Gonzalez-Gay MA, Lopez-Nevot MA, Torres B, et al. (2005) Association of a functional single-nucleotide polymorphism of PTPN22, encoding lymphoid protein phosphatase, with rheumatoid arthritis and systemic lupus erythematosus. Arthritis Rheum 52: 219-224.
Non-Patent Document No. 22: Mori M, Yamada R, Kobayashi K, Kawaida R, Yamamoto K (2005) Ethnic differences in allele frequency of autoimmune-disease-associated SNPs. J Hum Genet 50: 264-266.
Non-Patent Document No. 23: Ikari K, Kuwahara M, Nakamura T, Momohara S, Hara M, et al. (2005) Association between PADI4 and rheumatoid arthritis: a replication study. Arthritis Rheum 52: 3054-3057.
Non-Patent Document No. 24: Kang CP, Lee HS, Ju H, Cho H, Kang C, et al. (2006) A functional haplotype of the PADI4 gene associated with increased rheumatoid arthritis susceptibility in Koreans. Arthritis Rheum 54: 90-96.
Non-Patent Document No. 25: Barton A, Bowes J, Eyre S, Spreckley K, Hinks A, et al. (2004) A functional haplotype of the PADI4 gene associated with rheumatoid arthritis in a Japanese population is not associated in a United Kingdom population. Arthritis Rheum 50: 1117-1121.
Non-Patent Document No. 26: Caponi L, Petit-Teixeira E, Sebbag M, Bongiorni F, Moscato S, et al. (2005) A family based study shows no association between rheumatoid arthritis and the PADI4 gene in a white French population. Ann Rheum Dis 64: 587-593.
Non-Patent Document No. 27: Schellekens GA, de Jong BA, van den Hoogen FH, et al: Citrulline is an essential constituent of antigenic determinants recognized by rheumatoid arthritis-specific autoantibodies. J. Clin. Invest. 101:273-281, 1998
Non-Patent Document No. 28: Schellekens GA, Visser H, de Jong BA et al: The diagnostic properties of rheumatoid arthritis antibodies recognizing a cyclic citrullinated peptide. Arthritis Rheum. 43: 155-163, 2000

### DISCLOSURE OF THE INVENTION

### PROBLEM TO BE SOLVED BY THE INVENTION

It is believed that a large number of RA genetic factors in Japanese are yet to be found. Under the circumstances, the present invention aims at searching for novel genetic factors for RA by performing a large-scale GWAS in Japanese.

It is another object of the present invention to find out autoantibodies other than anti-CCP antibody which are useful in diagnosing anti-CCP antibody-negative RA patients.

### MEANS TO SOLVE THE PROBLEM

RA is a systemic disease that is the most common cause of adult chronic inflammatory arthritis. Although several genetic factors have been identified as risk factors for RA, they account for only a small part of the whole group of predicted genetic factors. The present inventors have conducted a large-scale case-control study in Japanese using a total of 225,079 single nucleotide polymorphisms (SNPs) as markers in an attempt to identify novel genetic risk factors for RA. The case-control study consisted of two sample sets for screening and two sample sets for result confirmation. The numbers of samples (RA patients vs healthy controls) were as follows: 658 cases vs 934 controls and 332 cases vs 307 controls in the screening sets, and 874 cases vs 855 controls and 1264 cases vs 948 controls in the result confirmation sets. As a result, the present inventors have found that a SNP (rs2000811) in intron 2 of myelin basic protein (MBP) gene located on the long arm of chromosome 18 is a novel risk factor for RA. Ap value in meta-analysis was 2.7*10^-8 and an odds ratio was 1.23 (with a 95% confidence interval of 1.14-1.32). The expression of MBP gene correlated with the SNP in intron 2. Further, the inventors have found that MBP protein is highly expressed in the synovial membrane of RA patients which is the main target of RA inflammation. The titers of autoantibodies to human brain-derived MBP protein (anti-MBP antibodies) in sera were investigated by ELISA, revealing that they were significantly higher in the group of RA patients than in the group of healthy people and in the group of patients with other connective tissue diseases (p value <0.001). In order to more closely investigate the protein to be recognized by anti-MBP antibodies, the present inventors prepared a recombinant MBP protein and citrullinated it *in vitro.* Using the citrullinated recombinant MBP protein and non-citrullinated recombinant MBP protein, correlations of antibody titers were examined by ELISA. As a result, it was found that antibodies reacting with human brain-derived MBP strongly correlated with citrullinated MBP. In conclusion, the present inventors have identified myelin basic protein as a novel genetic factor for RA by combining genetic methods with immunological methods.

The invention is defined by the appended claims. A summary of the present disclosure is as described below.
(1) A method of testing for rheumatoid arthritis, comprising detecting an autoantibody to myelin basic protein in a biological sample from a subject.
(2) A test kit for rheumatoid arthritis, comprising myelin basic protein.
(3) A diagnostic marker for rheumatoid arthritis, comprising an antibody to myelin basic protein.
(4) A method of judging the risk to develop rheumatoid arthritis, comprising identifying the single nucleotide polymorphism of a nucleotide present in the myelin basic protein gene of a subject or identifying the single nucleotide polymorphism of a nucleotide that is in linkage disequilibrium with the first mentioned nucleotide.
(5) The method of (4) above, wherein the single nucleotide polymorphism of a nucleotide present in the myelin basic protein gene is rs2000811 in the SNP database of the National Center for Biotechnology Information (NCBI), the United States.
(6) The method of (5) above, wherein susceptibility to rheumatoid arthritis is judged high when the nucleotide of the single polymorphism of rs2000811 is T in at least one allele or when the genotype of the single polymorphism of rs2000811 is C/T or T/T.
(7) A kit for judging the risk to develop rheumatoid arthritis, comprising nucleic acid probes and/or nucleic acid primers capable of detecting the single nucleotide polymorphism of a nucleotide present in the myelin basic protein gene of a subject or the single nucleotide polymorphism of a nucleotide that is in linkage disequilibrium with the first mentioned nucleotide.
(8) A method of screening for a substance effective as a prophylactic and/or therapeutic for rheumatoid arthritis, comprising adding a test substance to a myelin basic protein gene-expressing cell and then determining the expression level of the myelin basic protein gene or the gene product thereof.

### EFFECT OF THE INVENTION

According to the present invention, the association of rs2000811 with RA has been elucidated.

Further, according to the present invention, it was also shown that anti-MBP antibodies are RA markers. Anti-MBP antibodies are useful in diagnosis because they are not only highly specific but also test positive to some extent even in anti-CCP antibody- or rheumatoid factor-negative patients who have been difficult to diagnose.

The present specification claims priority of Japanese Patent Application No. 2011-95625.

### BRIEF DESCRIPTION OF THE DRAWINGS

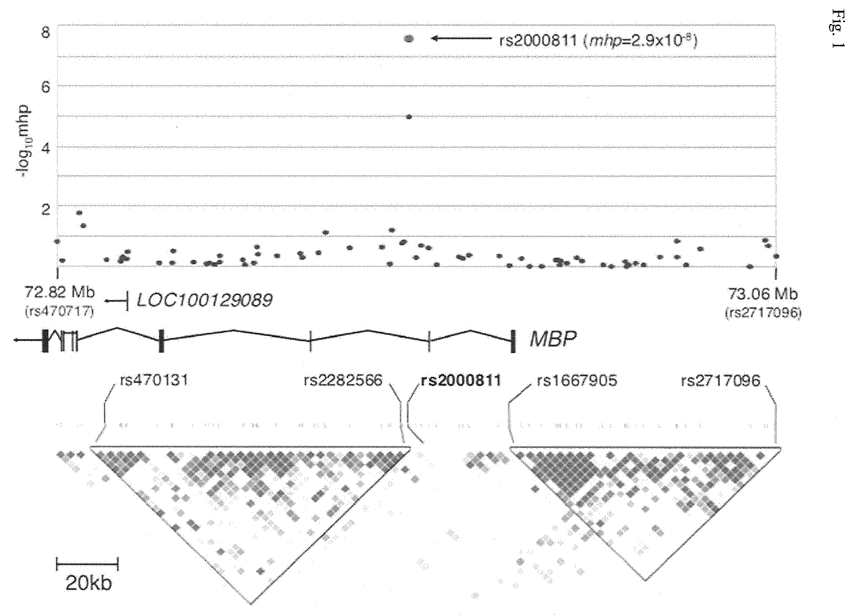

[Fig. 1] Results of association analyses of MBP gene locus at chromosome 18q23
   p values obtained from association analyses in SNPs located between rs470131 and rs2717096 are plotted in logarithm. Arrows indicate the orientations of genes. Red circle indicates the results of 4 sample sets for rs2000811. Blue dots indicate the results of 2 sample sets. Triangles show the results of linkage disequilibrium.

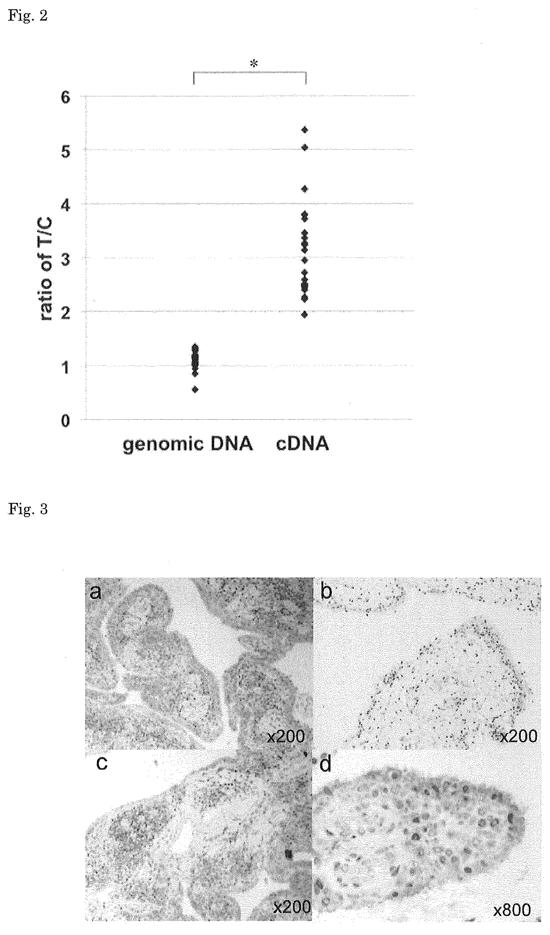

[Fig. 2] Allele-specific MBP transcription by allele-specific real-time PCR
   Amounts of mRNA precursors immediately after transcription from MBP gene were compared between alleles of rs2000811. The analysis was performed with RNA extracted from cells with hetropolymorphism. DNA from those cells was used as control, on the assumption that it was a 1:1 mixture of the two alleles.
[Fig. 3] Immunohistochemistry of the MBP protein in human synovial membrane using monoclonal anti-MBP antibody
   A) RA patient-derived synovial membrane strongly expressed MBP protein along the synovial lining layer. B) MBP protein was not expressed in osteoarthritis patients. C) The expression of MBP protein in RA patients' synovial membrane was weak around follicules of infiltrated lymphocytes. D) MBP protein was localized in the plasma membrane of synoviocytes.

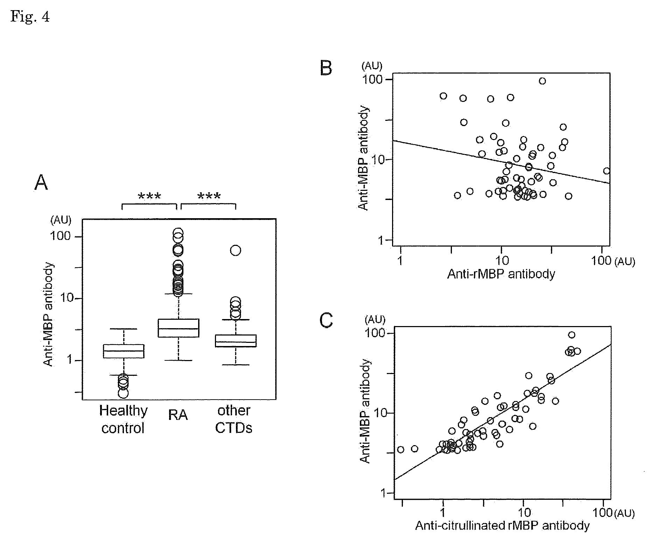

[Fig. 4] Quantification of circulating anti-MBP antibodies
   A) Comparison between healthy controls, RA patients, and patients with other connective tissue diseases. *** indicates *p* values smaller than 0.001. B) Correlation of autoantibody titers between human brain-derived MBP protein and recombinant MBP protein. C) Correlation of autoantibody titers between human brain-derived MBP protein and citrullinated recombinant MBP protein.

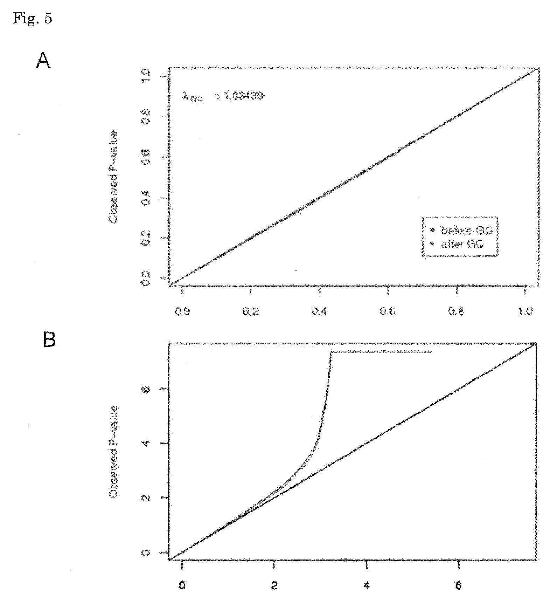

[Fig. 5] QQ plot showing comparison between observed and expected *p*-values
   A) Vertical and horizontal axes indicate observed and expected *p*-values, respectively. B) Both p-values are expressed in logarithm.
   The analysis using the genomic control method for determining the presence or absence of population stratification showed no significant effect of population stratification.
[Fig. 6] Linkage disequilibrium (LD) block of the 156-kb region comprising MBP gene
   LD map was generated with Haploview software on SNPs with allele frequencies between 0.05 and 0.95.
[Fig. 7] Allelic difference in MBP transcription
   Human B-lymphoblastoid cell lines immortalized by EBV were obtained from PSC. RNA was extracted for each genotype of rs2000811 and reverse-transcribed to cDNA (CC type: 50 persons; TC type: 50 persons; TT type: 49 persons). The transcription of MBP (Hs00921943-m1, Applied Biosystems Inc., Foster City, CA) was quantified by real time PCR using β-glucuronidase (Hs99999908_m1, Applied Biosystems Inc., Foster City, CA) as an endogenous reference. The ΔΔCT method was used to calculate transcription levels, and Jonckheere-Terpstra test was used for the analysis.

### BEST MODES FOR CARRYING OUT THE INVENTION

Hereinbelow, the present invention will be described in detail.

The present invention provides a method of testing for rheumatoid arthritis, comprising detecting an autoantibody to myelin basic protein in a biological sample from a subject.

In the present specification, the expression "testing for rheumatoid arthritis" encompasses those tests for judging whether the risk to develop rheumatoid arthritis (RA) is high or low in a subject and, when the subject is already affected by RA, those tests for conducting defmitive diagnosis of RA.

Specific examples of biological samples from subjects include, but are not limited to, blood (e.g., whole blood, serum, plasma, and extracellular fluid from plasma exchange), skin, oral mucosa, tissue or cell collected or excised by surgery, and body fluid collected for such purposes as testing (e.g., saliva, lymph, respiratory mucosa, sperm, sweat, and urine). As a biological sample, serum is preferable.

Myelin basic protein (MIM: 159430; OMIM database) is a protein consisting of 169 amino acids with a molecular weight of about 18000. MBP is a major constituent of the myelin sheath of neuronal cells. It is assumed that MBP is associated with the neurodegenerative disease multiple sclerosis (hereinafter, abbreviated to "MS"), because i) autoantibodies to MBP are found in blood and spinal fluid from MS patients and ii) injection of MBP to mice induces a condition similar to MS. On the other hand, no mutation in MBP gene has been found in MS patients. A number of transcription variants are transcribed from MBP gene and they are translated into proteins, which are roughly classified into classic MBP that constitutes the myelin sheath and Golli MBP that is expressed in oligodendrocytes and peripheral lymphocytes. Although the function of Golli MBP is largely unknown, it is suggested that Golli MBP may be involved in the regulation of intracellular Ca concentration and also involved in the differentiation of immune cells.

In the test method of the present invention, an autoantibody to MBP is detected in a biological sample from a subject.

For detecting autoantibodies to MBP, ELISA and Western blotting is preferably used. The MBP used for quantifying autoantibodies is preferably derived from human, and more preferably derived from human brain. MBP may be extracted from *in vivo* samples by biochemical techniques or may be prepared by genetic recombination techniques. Alternatively, a commercial MBP such as recombinant human MBP (Genscript, Piscataway, NJ) may be used. MBP may or may not be citrullinated. The citrullination of MBP may be performed by known methods using PAD (Lundberg K, Kinloch A, Fisher BA, Wegner N, Wait R, et al. (2008) Antibodies to citrullinated alpha-enolase peptide 1 are specific for rheumatoid arthritis and cross-react with bacterial enolase. Arthritis Rheum 58: 3009-3019).

When the concentration of autoantibody to MBP is 2.74 AU, it can be judged that the subject is very likely to develop RA or is already affected by RA. Alternatively, when the concentration of autoantibody to MBP is 2.16 AU or more, the result is regarded as positive and the final judgment of RA may be made after taking into consideration the results from other test methods (e.g., the rheumatoid factor (RF) test, the anti-CCP antibody measuring method (an autoantibody measuring method using an antigen that is an artificially circularized, citrullinated peptide), clinical observation, etc.).

Autoantibodies to MBP are useful as RA diagnostic markers.

The subject of the test method according to the present invention is a patient who is suspected of developing RA or being affected by RA; if desired, the subject may be any human who has a conceivable risk to develop RA.

Further, the present invention provides a test kit for RA, comprising MBP.

The MBP is preferably derived from human, and more preferably derived from human brain. MBP may be extracted from *in vivo* samples by biochemical techniques or may be prepared by genetic recombination techniques. Alternatively, a commercial MBP such as recombinant human MBP (Genscript, Piscataway, NJ) may be used. MBP may or may not be citrullinated. The citrullination of MBP may be performed by known methods using PAD (Lundberg K, Kinloch A, Fisher BA, Wegner N, Wait R, et al. (2008) Antibodies to citrullinated alpha-enolase peptide 1 are specific for rheumatoid arthritis and cross-react with bacterial enolase. Arthritis Rheum 58: 3009-3019). MBP protein extracted from *in vivo* samples by biochemical techniques, recombinant MBP protein, or citrullinated forms of these proteins is preferably bound to ELISA plates.

The test kit of the present disclosure may further comprise enzyme-labeled antibodies (e.g., alkaline phosphatase-labeled anti-human IgG polyclonal antibody (goat)), substrate solutions for enzymes (e.g., BCIP/NBT), reaction buffers, washing buffers, quenching solutions, standard solutions, positive controls, negative controls, and manuals describing criteria for judgment of RA, operational procedures, etc.

Further, the present invention provides a method of judging the risk to develop RA, comprising identifying the single nucleotide polymorphism of a nucleotide present in the MBP gene of a subject or identifying the single nucleotide polymorphism of a nucleotide that is in linkage disequilibrium with the first mentioned nucleotide. The single nucleotide polymorphism of a nucleotide present in the MBP gene is preferably rs2000811 in the SNP database of the National Center for Biotechnology Information (NCBI), the United States. When the nucleotide of the single polymorphism of rs2000811 is T in at least one allele or when the genotype of the single polymorphism of rs2000811 is C/T or T/T, susceptibility to rheumatoid arthritis can be judged high.

The nucleotide of the polymorphic site of rs2000811 may be represented as the 1102^{nd} nucleotide (y=C/T) in the nucleotide sequence as shown in SEQ ID NO: 1. The nucleotide sequence as shown in SEQ ID NO: 1 exists in the second intron of MBP gene on human chromosome 18.

The polymorphism which is in linkage disequilibrium with rs2000811 is preferably a polymorphism in the LD blocks of rs2000811.

When the D' value between SNPs is large, the SNPs are believed to be in linkage disequilibrium (Barrett JC, Fry B, Maller J, Daly MJ. Haploview: analysis and visualization of LD and haplotype maps. Bioinformatics. 2005;21(2):263-265.; Gabriel SB, Schaffner SF, Nguyen H, et al. The structure of haplotype blocks in the human genome. Science. 2002;296(5576):2225-2229). Therefore, the polymorphism in linkage disequilibrium with rs2000811 is, for example, such a polymorphism that the D' value between rs2000811 and itself is more than 0.8.

LD blocks may be determined with Haploview software (Barrett JC, Fry B, Maller J, Daly MJ. Haploview: analysis and visualization of LD and haplotype maps. Bioinformatics. 2005;21(2):263-265) by the method of Gabriel et al. (Gabriel SB, Schaffner SF, Nguyen H, et al. The structure of haplotype blocks in the human genome. Science. 2002;296(5576):2225-2229).

As an example of polymorphisms in the LD blocks of rs2000811, rs9958028 may be given. When the nucleotide of the single nucleotide polymorphism of rs9958028 is G in at least one allele, or when the genotype of the single nucleotide polymorphism of rs9958028 is A/G or G/G, susceptibility to RA can be judged high.

The nucleotide of the polymorphic site of rs9958028 may be represented as the 501^{st} nucleotide (r=A/G) in the nucleotide sequence as shown in SEQ ID NO: 2. The nucleotide sequence as shown in SEQ ID NO: 2 exists in the second intron of MBP gene on human chromosome 18.

In the present specification, single nucleotide polymorphism (SNP) is expressed in rs number which is a reference SNP ID number assigned in dbSNP (the SNP database of NCBI). The position of nucleotide is based on build36 which is a genome database of NCBI.

The SNP to be identified may be rs2000811 alone or a combination of rs2000811 and other SNP. Specific examples of other SNP include, but are not limited to, rs9958028.

Identification of the nucleotide of the polymorphic site (i.e., determination of nucleotide species) may be performed by known methods for single nucleotide polymorphism analysis. Specific examples of such methods include, but are not limited to, direct sequencing, the RFLP method, the PCR-SSCP method, allele-specific oligonucleotide hybridization, TaqMan PCR, the invader method, the MALDI-TOF/MS method, the molecular beacon method, RCA, the UCAN method, and nucleic acid hybridization using DNA chips or DNA microarrays.

For identification of the nucleotide of the polymorphic site, genomic DNA may be extracted from a biological sample of a subject. Examples of biological samples include, but are not limited to, subject's blood (e.g., whole blood, serum, plasma, and extracellular fluid from plasma exchange), skin, oral mucosa, tissue or cell collected or excised by surgery, and body fluid collected for such purposes as testing (e.g., saliva, lymph, respiratory mucosa, sperm, sweat, and urine). As a biological sample, plasma is preferable. It is possible to extract genomic DNA from a biological sample using a commercial DNA extraction kit. Subsequently, if necessary, DNA fragments comprising a polymorphic site are isolated. This isolation of DNA fragments may typically be performed by PCR using primers capable of hybridizing to the DNA fragment comprising a polymorphic site, with genomic DNA or RNA being used as a template.

The present disclosure also provides a kit for judging the risk to develop rheumatoid arthritis, comprising nucleic acid probes and/or nucleic acid primers capable of detecting the single nucleotide polymorphism of a nucleotide present in the myelin basic protein gene of a subject or the single nucleotide polymorphism of a nucleotide that is in linkage disequilibrium with the first mentioned nucleotide.

The single nucleotide polymorphism of a nucleotide present in the myelin basic protein gene of a subject and the single nucleotide polymorphism of a nucleotide that is in linkage disequilibrium with the first mentioned nucleotide are as described above.

The primers and/or probes contained in the kit of the present invention are preferably oligonucleotides with a length of at least 15 nucleotides. When oligonucleotides are to be used as primers, their length is usually 15 bp to 100 bp and preferably 17 bp to 30 bp. Primers are not particularly limited as long as they are capable of amplifying at least a part of a DNA fragment containing the above-described polymorphic site. The length of DNA fragment which can be amplified with primers is usually 15-1000 bp, preferably 20-500 bp, and more preferably 20-200 bp. When oligonucleotides are to be used as probes, their length is usually 15-500 bp and preferably 30-500 bp. Probes are not particularly limited as long as they are capable of hybridizing to a DNA fragment containing the above-described polymorphic site. The length of DNA fragment to which probes can hybridize is usually 16-500 bp, preferably 20-200 bp, and more preferably 20-500 bp. Any person skilled in the art could appropriately design such primers and probes based on nucleotide sequence information about DNA regions surrounding the polymorphic site. Such primers and/or probed may be labeled in advance (with radioisotope, fluorescent dye or the like). Probes may be immobilized on a solid phase such as a substrate.

The kit of the present disclosure may further comprise hybridization reagents, agents for detecting the label of primers and/or probes, buffers, manuals describing criteria for judgment of the risk to develop RA and the method of using the kit, and ELISA plates to which MBP protein extracted from *in vivo* sample by biochemical techniques, recombinant MBP protein, or citrullinated forms of such MBP proteins are bound.

Further, the present disclosure provides a method of screening for a substance effective as a prophylactic and/or therapeutic for rheumatoid arthritis, comprising adding a test substance to a myelin basic protein gene-expressing cell and then determining the expression level of the myelin basic protein gene or the gene product thereof.

The myelin basic protein gene-expressing cell may be derived from any organism as long as the expression of myelin basic protein can be observed at the protein level or the nucleic acid level and examples include, but are not limited to, cells derived from mammals such as human, pig, monkey, chimpanzee, dog, cattle, rabbit, rat, and mouse. It is preferable to use human-derived cells (e.g., neuronal cells).

The test substance may be any substance. Examples of the test substance include, but are not limited to, proteins, peptides, vitamins, hormones, polysaccharides, oligosaccharides, monosaccharides, low molecular weight compounds, nucleic acids (DNA, RNA, oligonucleotide, mononucleotide, etc.), lipids, natural compounds other than those listed above, synthetic compounds, plant extracts, fractions from plant extracts, and mixtures thereof.

Quantification of the expression level of MBP gene or the gene product thereof may be performed by determining the amount of the transcription product of MBP gene or the activity of the transcription product.

The amount of the transcription product may be determined by performing quantitative PCR analysis on the cDNA, as obtained through reverse transcription of RNA in the sample, using a primer set for amplifying an MBP gene-specific sequence. If applicable, Northern blotting with a probe specific to MBP gene may be performed. Alternatively, the transcription product may be quantified by using DNA chips.

The amount and/or activity of the transcription product of MBP gene can typically be detected by immunoassay, quantification of enzyme activities, or binding assay. These assays may be performed by using a label (e.g. enzymatic, fluorescent, radioactive, magnetic, or luminescent) that binds to anti-MBP antibody or a secondary antibody binding to anti-MBP antibody and determining the amount of binding between the above-described translation product and anti-MBP antibody.

### EXAMPLE

Hereinbelow, the present invention will be described in more detail with reference to the following Example. However, the present invention is not limited to this Example.

### [Example 1]

### Techniques

Four case-control sample sets (collections) were used. Two collections were used in GWAS for screening, and the other two were used for confirmation of the screening results. The number of samples (RA patients vs healthy controls) in each collection was as follows: 1^{st} collection for screening: 658 cases vs 934 controls; 2^{nd} collection for screening: 332 cases vs 307 controls; 3^{rd} collection for result confirmation: 874 cases vs 855 controls; and 4^{th} collection for result confirmation: 1264 cases vs 948 controls (Table 2). With respect to the 934 healthy controls in the 1^{st} collection, data publicly disclosed in JSNP were used. The samples were plasma samples taken from patients in Kyoto University, Dohgo Spa Hospital, Tokyo Women's Medical University, The University of Tokyo, Sagamihara National Hospital, and Aichi Cancer Center; also used were cell lines obtained from PSC. All patients satisfied the criteria of the ACR. Studies were approved by the ethical committees of relevant research organizations.

### Association Analysis:

For GWAS, Infinium chips of Illumina Inc. were used. With four different chips (Human-Hap300, CNV370-Duo, HumanHap550, and Human610-Quad), the present inventors focused on 277420 SNPs common to these four chips. DNA samples removed from analyses were with a call rate smaller than 90%, showed kinship with other samples, did not fall into the Japanese cluster on principal component analysis, and were suspected of possible contamination with other DNA. SNPs with a call rate greater than 95% and a minor allele frequency greater than 5% were selected. As a result, the numbers of remaining samples were 643 vs 934 in the 1^{st} collection and 327 vs 297 in the 2^{nd} collection. Regarding the SNP markers, a total of 225,079 markers were obtained. Association analysis was performed on each marker in the 1^{st} and 2^{nd} collections independently. Those SNPs that showed *p*<0.005 in both collections and *p*-values smaller than 0.0001 in meta-analysis of the 1^{st} and 2^{nd} collections were selected. Except for those regions where association is known in Japanese RA cases, a SNP with the smallest *p*-value in meta-analysis was selected from a plurality of markers that were found in one LD block. For the thus selected SNPs, association was confirmed in the 3^{rd} and 4^{th} collections.

### Determination of the Nucleotide Sequence of MBP Region:

After finding association of MBP region, the present inventors performed sequencing of the promoter region and the amino acid-encoding exon regions of the MBP gene in 84 healthy control DNAs.

### Bioinformatics:

Genome sequence alignment of 14 placental mammals was obtained from a publicly disclosed database. Transcriptional regulatory elements were searched for in regions comprising SNPs of interest.

### Quantification of Allele-Specific MBP Transcription:

Quantification of allele-specific MBP transcription was performed as already known (e.g., Suzuki A, Yamada R, Kochi Y, Sawada T, Okada Y, et al. (2008) Functional SNPs in CD244 increase the risk of rheumatoid arthritis in a Japanese population. Nat Genet 40: 1224-1229).

Briefly, human B-lymphoblastoid cell lines immortalized by EBV were obtained from PSC. DNA and RNA were extracted by standard procedures from 22 cell lines that were heterozygous (TC type) for rs2000811 alleles. The amounts of mRNA precursors were compared between alleles using real time PCR probes for SNP typing.

DNA samples from homozygous (CC type and TT type) cell lines were mixed at ratios of 2:1, 3:2, 1:1, 2:3, 1:2, 1:3, 1:4, and 1:6 to draw standard curves for the calculation of RNA quantity.

### Immunohistochemistry:

Joint synovial tissue samples were obtained from 23 RA patients and 5 control patients in Department of Diagnostic Pathology and Department of Orthopaedic Surgery, both in Kyoto University Hospital. The tissue samples were embedded in paraffm and sectioned to give a thickness of 3 µm with standard techniques. Each section was mounted on a glass slide coated with APS. Immunohistochemical staining of MBP was performed by the standard ABC method.

The sections were incubated overnight at 4°C with a mouse anti-MBP monoclonal antibody diluted at 1:100 in PBS buffer. The sections were then incubated with biotinylated horse anti-mouse IgG antibody for 40 minutes, followed by incubation with peroxidase-conjugated streptavidin at room temperature for 50 minutes.

The coloring reaction was performed with 0.3 mg/ml diaminobenzidine and 0.003% H₂O₂ dissolved in 50 mM Tris-HCl buffer (pH 7.6).

Each section was counterstained with HE. Evaluation of MBP expression was performed by a blind test by two rheumatologists and a pathologist. The results were analyzed by Fisher's exact test.

### ELISA:

The presence or absence of anti-MBP antibodies was examined by ELISA on serum samples from 323 RA patients, 133 healthy controls, and 162 patients with other connective tissue diseases (SLE: 38 cases; Sjögren's syndrome: 25 cases; scleroderma: 25 cases; Behcet's disease: 20 cases; mixed connective tissue disease: 20 cases; dermatomyositis or polymyositis: 19 cases; and vasculitis: 15 cases). As an antigen, a human brain-derived MBP protein (Sigma, St. Louis, MO) was used. Further, a recombinant human MBP protein (Genscript, Piscataway, NJ.) was prepared and citrullinated with rabbit skeleton PAD (Sigma, St. Louis, MO) (Lundberg K, Kinloch A, Fisher BA, Wegner N, Wait R, et al. (2008) Antibodies to citrullinated alpha-enolase peptide 1 are specific for rheumatoid arthritis and cross-react with bacterial enolase. Arthritis Rheum 58: 3009-3019). The reactivity of autoantibodies was quantified using non-citrullinated recombinant MBP protein and citrullinated recombinant MBP protein.

### Results

### Association Analysis:

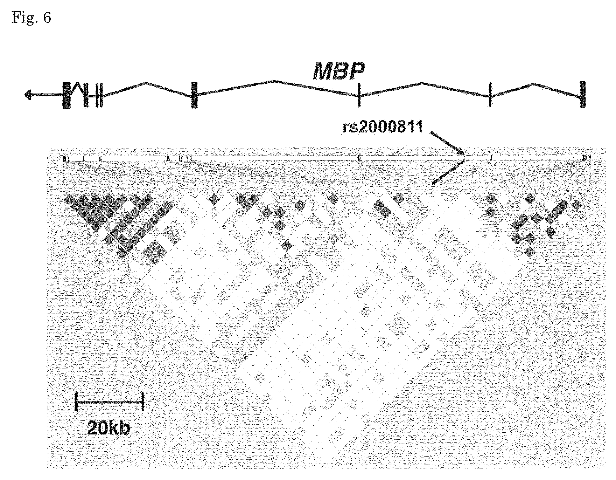

Four case-control collections totaling 3128 cases and 3044 controls were used for GWAS analysis (Table 2). Markers were selected based on the results of GWAS. As a result, 225,079 markers common to the used chips were judged to have no problem for use in assays. Association analysis was performed on these 225,079 markers. Mean call rate was 99.5% in the 1^{st} collection and 99.8% in the 2^{nd} collection. No lowering of *p*-value due to stratification of samples was observed (λ=1.03, Fig. 5). As a result of meta-analysis of the 1^{st} and 2^{nd} collections, two regions showed low *p*-values and they were HLA (*mhp*=3.6x10⁻³¹) and *PADI4* (*mhp*=3.0x10⁻⁵) already known to have association with Japanese RA cases. It was believed that these results demonstrate the correctness of our data (Table 1). Since 10 SNPs in 5 regions were left through SNP selection criteria, the SNP with the smallest *p*-value was selected from each of these regions (5 SNPs in total) and subjected to typing in the 3^{rd} and 4^{th} collections. Of these 5 SNPs, rs2000811 alone reproduced association in the 3^{rd} collection (*p*-value 0.023) and the 4^{th} collection (*p*-value 0.0041). In the total of 4 collections, a *p*-value was 4.0x10⁻⁸ and an odds ratio was 1.23 (with a 95% confidence interval of 1.14-1.32); in meta-analysis, *mhp* was 2.7x10⁻⁸ and an odds ratio was 1.23 (with a 95% confidence interval of 1.14-1.32). The location of rs2000811 was found to be in the second intron of MBP gene on chromosome 18. When linkage disequilibrium (LD) of this region was evaluated with the results of SNP typing in the 1^{st} and 2^{nd} collections, it was found that this SNP (rs2000811) is located in a 18 kb segment flanked by two LD blocks contained in a 218 kb region (Fig. 1). In addition to MBP gene, a gene *LOC100129089* was also found in this 218 kb region. Since rs2000811 was not in strong linkage disequilibrium with other SNPs on GWAS chips, it was believed that MBP gene is associated with RA. However, it is not known whether rs2000811 per se is a cause of different susceptibility to RA, or whether there is another causative variation and rs2000811 is simply a marker in LD with that variation. Therefore, the present inventors performed sequencing of the promoter region and the amino acid-encoding exon regions of the MBP gene in 84 healthy control DNAs. As a result, 66 SNPs and 3 deletion mutations were found but none of them were in strong LD with rs2000811 (Fig. 6). Association of MBP Gene Expression with rs2000811

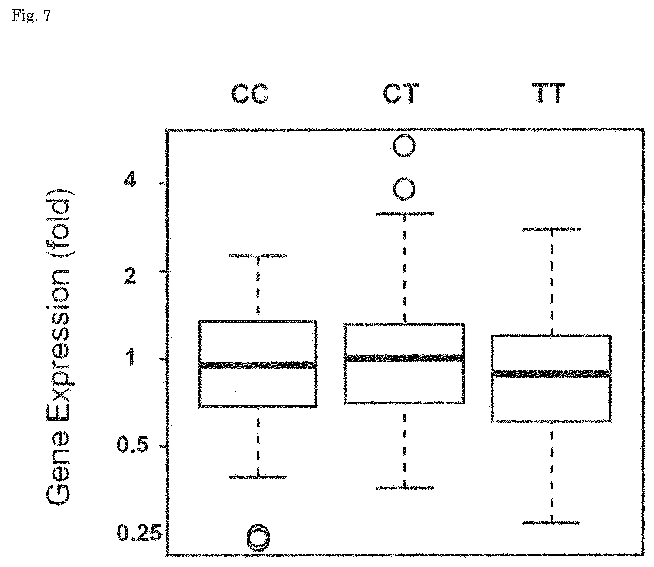

Subsequently, the present inventors examined whether rs2000811-containing sequences might be involved in transcription. DNA and RNA were extracted from immortalized lymphoblastoid cell lines in 22 healthy controls who were heterozygous for rs2000811 alleles, and RNA was reverse transcribed to cDNA. Analysis of allele-specific transcription using the DNA and cDNA revealed that the polymorphism of rs2000811 associated with RA is involved in elevated expression of MBP gene (Fig. 2). MBP is roughly classified into two transcription products and proteins: classic MBP and Golli MBP. cDNA samples obtained from lymphoblastoid cell lines in a total of 149 healthy controls as separated for each polymorphism of rs2000811 were studied, but no association was observed between classic MBP expression and rs2000811 polymorphism (Fig. 7). Hence, association of rs2000811 with Golli MBP expression was assumed to be the case.

When known transcriptional regulatory elements were searched through database, no such element was found in the region containing rs2000811. This region was also found to have comparatively low interspecies conservation among placental mammals.

### Examination of MBP Protein Expression in the Synovial Membrane of RA Patients:

Inflammation in RA is mainly observed in the joint synovial membrane. The present inventors obtained synovial membrane tissue samples from 23 RA patients and 5 non-RA patients with such diseases as osteoarthritis, and performed immuno-staining with anti-MBP antibodies. The results revealed a highly frequent and strong expression of MBP protein in the synovial membrane of RA patients (Fig. 3).

### Quantification of Anti-MBP Antibodies in RA Patients:

It is known that anti-MBP antibodies play an important role in multiple sclerosis, an autoimmune neurodegenerative disease. The present inventors quantified the anti-MBP antibodies in serum samples obtained from 323 RA patients, 133 healthy controls, and 162 patients with other connective tissue diseases. Anti-MBP antibody titers in RA patients were higher than those in healthy controls and patients with other connective tissue diseases (*p*<0.001, Fig. 4A). There was no apparent association between anti-MBP antibody titers and rs2000811 genotypes. In order to investigate more closely the antigen which anti-MBP antibodies recognizes, the present inventors prepared recombinant MBP proteins and citrullinated these proteins. The thus citrullinated recombinant MBP proteins and non-citrullinated MBP proteins were assayed by ELISA. As a result, no correlation was found between recombinant MBP proteins and human brain-derived MBP (r = -0.19, Fig. 4B). When citrullinated recombinant MBP protein was used, strong correlation was observed in autoantibody titers (r=0.88, Fig. 4C). Briefly, anti-MBP antibodies strongly reacted with citrullinated recombinant MBP (Fig. 4B and 4C) and, hence, were believed to recognize citrullinated MBP as the main target.

### Discussion

In the study described above, the present inventors elucidated the association between RA and a SNP located in the second intron of MBP gene on chromosome 18 by GWAS-based analyses. This SNP was flanked with two relatively weak LD blocks. Determination of the nucleotide sequence of the MBP region revealed that no genetic variation in strong LD with this SNP is present in the exon regions or the promoter region. The present inventors found that expression of MBP gene is associated with this SNP, but failed to show any relationship between this SNP-containing sequence and known transcriptional regulatory elements. For this reason, it is unknown whether rs2000811 per se or a genetic variation in LD with rs2000811 (which could not be found in the study described above) is the cause to alter the expression of MBP gene. Examination of the nucleotide sequence of the 18 kb region between the two LD blocks flanking rs2000811 might elucidate a variation as the true cause. It has not been reported to date that this region is associated with RA in a European population. Since the susceptibility to RA varies greatly depending on race, this region may not be associated in a European population but confirmation of the result for each race is important for understanding the pathology that underlies the disease. MBP is classified into classic MBP which is found in neuronal cells and Golli-MBP which is also found in hematopoietic cells, and rs2000811 is located in the second intron in the Golli-MBP encoding region. Since only a small number of RA cases present with neurologic symptoms, the susceptibility to RA may be associated with Golli-MBP.

Further, the present inventors showed that MBP protein is expressed strongly and at high frequency in the synovial membrane of RA patients. Expression of MBP protein on the cell surface of synovial membrane may be suggesting that anti-MBP antibodies cause inflammation targeting at synovial membrane cells.

The present inventors have also shown that anti-MBP antibodies are RA markers. Many, but not all, of anti-MBP antibodies seem to recognize citrullinated MBP. Although anti-MBP antibodies were not associated with SNPs, some SNPs may be involved in post-translational modifications. The study of the present inventors is the first to show not only the genetic association of MBP with RA but also the expression of MBP gene, the expression of MBP protein, and the occurrence of autoantibodies to MBP.

**Table 1. Association of MBP, HLA and PADI4 loci with rheumatoid arthritis in the Japanese population.**

| Chr | dbSNPID | Gene | Allele | DNA Collection | | Genotype counts | | | Success rate | HWE*p* | RAF** | *p*-value | OR (95%CI) | *mhp**** |
|---|---|---|---|---|---|---|---|---|---|---|---|---|---|---|
| | | | Ref.(A1)/Var.(A2) | | | A1A1 | A1A2 | A2A2 | | | | | | |
| 18q23 | rs2000811 | *MBP* | C/T* | 1 | case | 203 | 303 | 136 | 99.8 | 0.25 | 0.45 | 0.0036 | 1.25(1.08-1.44) | |
| | | | | | control | 344 | 442 | 148 | 100 | 0.76 | 0.4 | | | |
| | | | | 2 | case | 95 | 152 | 79 | 99.7 | 0.24 | 0.48 | 5.7x10⁻⁴ | 1.49(1.19-1.87) | |
| | | | | | control | 120 | 131 | 46 | 100 | 0.31 | 0.38 | | | |
| | | | | 3 | case | 283 | 392 | 182 | 98.1 | 0.034 | 0.44 | 0.023 | 1.17(1.02-1.34) | |
| | | | | | control | 298 | 404 | 134 | 97.8 | 0.88 | 0.4 | | | |
| | | | | 4 | case | 393 | 622 | 233 | 98.7 | 0.63 | 0.44 | 0.0041 | 1.19(1.06-1.35) | |
| | | | | | control | 341 | 451 | 141 | 98.4 | 0.68 | 0.39 | | | |
| | | | | 3+4 | case | 676 | 1014 | 415 | 985 | 0.32 | 0.44 | 3.0x10⁻⁴ | 1.18(1.08-1.30) | |
| | | | | | control | 639 | 855 | 275 | 98.1 | 0.69 | 0.40 | | | |
| | | | | pooled | case | 974 | 1469 | 630 | 98.9 | 0.078 | 0.44 | 4.0x10⁻⁸ | 1.23(1.14-1.32) | 2.7x10⁻⁸ |
| | | | | | control | 1103 | 1428 | 469 | 98.9 | 0.85 | 0.39 | | | |
| 6p21 | rs2516049 | *HLA-DRB1* | T/C* | 1 | case | 249 | 300 | 94 | 100 | 0.81 | 0.38 | 32x10⁻²¹ | 2.13(1.82-2.49) | |
| | | | | | control | 558 | 335 | 41 | 100 | 0.3 | 0.22 | | | |
| | | | | 2 | case | 118 | 171 | 37 | 99.7 | 0.033 | 0.38 | 95x10⁻¹² | 2.35(1.82-3.04) | |
| | | | | | control | 187 | 99 | 11 | 100 | 0.64 | 0.2 | | | |
| | | | | 3 | case | 367 | 471 | 131 | 99.9 | 0.3 | 0.38 | 3.6x10⁻³¹ | 2.18(1.91-2.48) | 5.0x10⁻³¹ |
| | | | | | control | 745 | 434 | 52 | 100 | 0.26 | 0.22 | | | |
| 1p36 | rs2240335 | *PAD14* | C*/A | 1 | case | 148 | 313 | 178 | 99.4 | 0.65 | 0.48 | 0.0014 | 1.27(1.10-1.46) | |
| | | | | | control | 171 | 436 | 324 | 99.7 | 0.25 | 0.42 | | | |
| | | | | 2 | case | 80 | 153 | 93 | 99.7 | 0.28 | 0.48 | 0.0055 | 1.38(1.10-1.73) | |
| | | | | | control | 47 | 143 | 106 | 99.7 | 0.92 | 0.4 | | | |
| | | | | 3 | case | 228 | 466 | 271 | 99.5 | 0.32 | 0.48 | 3.0x10⁻⁵ | 1.30(1.15-1.46) | 2.3x10⁻⁵ |
| | | | | | control | 218 | 579 | 430 | 99.7 | 0.34 | 0.41 | | | |

| | | | | | | | | | | | | | | |
|---|---|---|---|---|---|---|---|---|---|---|---|---|---|---|
| *risk allele for the disease, **risk allele frequency, and ****p*-value in meta-analysis using Cochran-Mantel-Haenszel test. | | | | | | | | | | | | | | |

**Table 2. Summary of the study populations used for the association analysis.**

| Sample set | | Number | Female (%) | Age (mean±SD) | Genotyping Method | Average Success Rate (%) | Autoantibody positivity (%) |
|---|---|---|---|---|---|---|---|
| RA | collection1 | 643 | 82.0 | 64.4±12.4 | Human-Hap300, Human CNV370-Duo | 99.5 | ACPA: 72.3, RF: 86.1 |
| | collection2 | 327 | 80.0 | 61.3±13.0 | Human610-Quad | 99.8 | ACPA: 82.0, RF: 86.1 |
| | collection3 | 874 | 85.5 | 62.6±11.6 | Taqman | - | N/A |
| | collection4 | 1264 | 83.0 | 59.7±11.9 | Taqman | - | N/A |
| | | | | | | | |
| Control | collection1 | 934 | N/A | N/A | HumanHap550 | N/A | |
| | collection2 | 297 | 68.7 | 58.8±13.2 | HumanHap550 | 99.9 | |
| | collection3 | 855 | 39.6 | 38.1±11.9 | Taqman | - | |
| | collection4 | 948 | 48.9 | 48.4±16.3 | Taqman | - | |

| | | | | | | | |
|---|---|---|---|---|---|---|---|
| Abbreviations were as follows; ACPA: antibodies to citrullinated peptide antigens, RF: rheumatoid factor, SD: standard deviation, N/A, not available. | | | | | | | |

### INDUSTRIAL APPLICABILITY

The present invention is applicable to diagnosis of RA. The present disclosure is also applicable to screening for substances effective for prevention and/or treatment of RA.

### SEQUENCE LISTING FREE TEXT

### <SEQ ID NO: 1>

SEQ ID NO: 1 shows a nucleotide sequence of 1602 bp comprising the polymorphic site of rs2000811 at position 1102 (y=C/T).
SEQ ID NO: 2 shows a nucleotide sequence of 1001 bp comprising the polymorphic site of rs9958028 at position 501 (r=A/G).

## Claims

1. A method of testing for rheumatoid arthritis, comprising identifying the single nucleotide polymorphism (SNP) of a nucleotide present in the myelin basic protein gene of a subject or identifying the SNP of a nucleotide that is in linkage disequilibrium with the first mentioned nucleotide, wherein the SNP of a nucleotide present in the myelin basic protein gene is rs2000811 in the SNP database of the National Center for Biotechnology Information (NCBI), the United States, and the SNP of a nucleotide that is in linkage disequilibrium with the first mentioned nucleotide is rs9958028.

2. The method according to claim 1, wherein it is judged that the subject is affected with rheumatoid arthritis or is highly susceptible to rheumatoid arthritis when the nucleotide of the single polymorphism of rs2000811 is T in at least one allele or when the genotype of the single polymorphism of rs2000811 is C/T or T/T.

3. Use of nucleic acid probes and/or nucleic acid primers for judging the risk to develop rheumatoid arthritis, wherein the nucleic acid probes and/or nucleic acid primers are capable of detecting the SNP of a nucleotide present in the myelin basic protein gene of a subject or the SNP of a nucleotide that is in linkage disequilibrium with the first mentioned nucleotide, wherein the SNP of a nucleotide present in the myelin basic protein gene is rs2000811 in the SNP database of the National Center for Biotechnology Information (NCBI), the United States, and the SNP of a nucleotide that is in linkage disequilibrium with the first mentioned nuelcotide is rs9958028.

## Patentansprüche

1. Verfahren zum Testen auf rheumatoide Arthritis, welches die Identifizierung des einzelnen Nucleotidpolymorphismus (single nucleotide polymorphism (SNP)) von einem Nucleotid, welches in dem Myelin-basisches-Protein-Gen eines Subjekts vorliegt oder Identifizierung des SNPs von einem Nucleotid, welcher in einem Kopplungsungleichgewicht mit dem ersten erwähnten Nucleotid ist, umfasst, wobei der SNP eines Nucleotids, welcher in dem Myelin-basisches-Protein-Gen vorhanden ist, rs2000811 in der SNP-Datenbank des National Center for Biotechnology Information (NCBI), Vereinigte Staaten, ist und der SNP eines Nucleotids, welcher im Kopplungsungleichgewicht mit dem ersten erwähnten Nucleotid ist, rs9958028 ist.

2. Verfahren gemäß Anspruch 1, wobei es zu beurteilen ist, dass das Subjekt durch rheumatoide Arthritis beeinträchtigt ist, oder hochanfällig für rheumatoide Arthritis ist, wenn das Nucleotid des einzelnen Polymorphismus von rs2000811 T in mindestens einem Allel ist, oder, wenn der Genotyp des einzelnen Polymorphismus von rs2000811 C/T oder T/T ist.

3. Verwendung von Nucleinsäuresonden und/oder Nucleinsäureprimern zur Beurteilung des Risikos, rheumatoide Arthritis zu entwickeln, wobei die Nucleinsäuresonden und/oder Nucleinsäureprimer fähig sind, den SNP eines Nucleotids, welcher in dem Myelin-basisches-Protein-Gen eines Subjekts vorhanden ist, oder den SNP eines Nucleotids, welcher im Kopplungsungleichgewicht mit dem ersten erwähnten Nucleotid ist, zu detektieren, wobei der SNP eines Nucleotids, welcher in dem Myelin-basisches-Protein-Gen vorhanden ist, rs2000811 in der SNP-Datenbank des National Center for Biotechnology Information (NCBI), Vereinigte Staaten, ist und der SNP eines Nucleotids, welcher im Kopplungsungleichgewicht mit dem ersten erwähnten Nucleotid ist, rs9958028 ist.

## Revendications

1. Procédé pour tester la présence de polyarthrite rhumatoïde, comprenant l'identification du polymorphisme d'un seul nucléotide (SNP) d'un nucléotide présent dans le gène de protéine basique myéline d'un sujet ou l'indentification du SNP d'un nucléotide qui est en déséquilibre de liaison avec le nucléotide mentionné en premier, le SNP d'un nucléotide présent dans le gène de protéine basique myéline étant rs200811 dans la base de données SNP du National Center for Biotechnology Information (NCBI) des États-Unis, et le SNP d'un nucléotide qui est en déséquilibre de liaison avec le nucléotide mentionné en premier étant rs9958028.

2. Procédé selon la revendication 1, dans lequel on juge que le sujet est atteint de polyarthrite rhumatoïde ou est hautement susceptible de développer une polyarthrite rhumatoïde lorsque le nucléotide du polymorphisme d'un seul nucléotide de rs200811 est T dans au moins un allèle ou lorsque le génotype du polymorphisme d'un seul nucléotide de rs200811 est C/T ou T/T.

3. Utilisation de sondes d'acide nucléique et/ou d'amorces d'acide nucléique pour évaluer le risque de développer une polyarthrite rhumatoïde, dans laquelle les sondes d'acide nucléique et/ou les amorces d'acide nucléique sont capables de détecter le SNP d'un nucléotide présent dans le gène de protéine basique myéline d'un sujet ou le SNP d'un nucléotide qui est en déséquilibre de liaison avec le nucléotide mentionné en premier, le SNP d'un nucléotide présent dans le gène de protéine basique myéline étant rs200811 dans la base de données SNP du National Center for Biotechnology Information (NCBI) des États-Unis, et le SNP d'un nucléotide qui est en déséquilibre de liaison avec le nucléotide mentionné en premier étant rs9958028.
